# EUROPEAN PATENT SPECIFICATION

(11) **EP 0 563 288 B1**
(45) Date of publication and mention of the grant of the patent: **03.03.1999**
(21) Application number: 92903516.0
(22) Date of filing: 19.12.1991
(51) Int. Cl.: A61K 39/102

(54) **IMMUNOPOTENTIATION OF VACCINES, PARTICULARLY SWINE PLEUROPNEUMONIA VACCINES**
IMMUNVERSTÄRKUNG VON IMPFSTOFFEN, BESONDERS VON IMPFSTOFFEN GEGEN SCHWEINEPLEUROPNEUMONIE
IMMUNOPOTENTIALISATION DE VACCINS, NOTAMMENT DE VACCINS CONTRE LA PLEUROPNEUMONIE PORCINE

(30) Priority: 19.12.1990 US 631009
(43) Date of publication of application: 06.10.1993
(73) Proprietor: PFIZER INC., New York, N.Y. 10017-5755 (US)
(72) Inventor: DAYALU, Krishnaswamy, Iyengar, Lincoln, NB 68506 (US); ZYGRAICH, Nathan, West Chester, PA 19380 (US)
(74) Representative: Simpson, Alison Elizabeth Fraser
(86) International application number: US9109641
(87) International publication number: WO9211023

(56) References cited:
- EP-A- 0 287 206
- EP-A- 0 354 628
- EP-A- 0 389 347
- EP-A- 0 453 024
- GB-A- 2 029 219
- Infection and Immunity, Vol. 57, No. 3, issued March 1989, DENEER et al., "Effect of Iron Restriction on the Outer Membrane Protein of Actinobacillus (Haemophilus) pleuropneumoniae" pp. 798-804, see entire document.
- Can. Vet. J., Vol. 26, issued 1985, HIGGINS et al., "Evaluation of a killed vaccine against porcine pleuropneumomia due to Haemophilus pleuropneumoniae", pp. 86-89, see entire document.
- J. Clinical Microbiology, Vol. 27, No. 6, issued June 1989, KAMP et al., "Serotype-Related Differences in Production and Type of Heat-Labile Hemolysin and Heat-Labile Cytotoxin of Actinobacillus (Haemophilus) Pleuropneumoniae", pp. 1187-1191, see the entire document.
- Infection and Immunity, Vol. 56, No. 8, issued August 1988, INZANA et al., "Virulence Properties and Protective Efficacy of the Capsular Polymer of Haemophilus (Actinobacillus) Pleuropneumoniae, Serotype 5", pp. 1880-1889, see entire document.

## Description

This invention relates generally to the field of veterinary vaccines. More specifically, this invention relates to novel vaccines and methods for enhancing the immune response to vaccines directed against diseases associated with animals infected by A. pleuropneumoniae.

### Background of the Invention

Actinobacillus (Haemophilus) pleuropneumoniae is a swine pathogen which causes an acute pneumonia with fibrinous pleuritis or chronic lung lesions in infected animals [T.N. Sebunya et al, J. Am. Vet. Med. Assoc., 182:1331-1337 (1983)].

The phenotype of A. pleuropneumoniae, like that of other pathogens, is influenced by many factors in the host environment during infection. One common host defense mechanism involves the withholding of essential bacterial nutrients, e.g. iron. While most iron in body fluids is located intracellularly, extracellular iron is tightly complexed with iron-binding and transport proteins such as transferrin and lactoferrin. Most pathogenic bacteria have developed high-affinity iron uptake systems to obtain this bound iron. These uptake systems usually involve two components, low molecular weight siderophores which chelate iron and iron repressible outer membrane proteins (IROMPS) which function as receptors of the ironsiderophore complexes [H. Deneer, Infection and Immunity, 57(3):798-804 (1989); V. Braun, Trends Biochem. Sci., 10:75-78 (1985); M.C. McIntosh et al, J. Bacteriol., 137:653-657 (1979); J.B. Neilands et al, Annu. Rev. Microbiol., 36:285-310 (1982)].

IROMPS from several pathogenic microorganisms have been shown to be immunogenic and, in many cases, show a significant degree of immunological cross-reactivity between the serogroups of a single species. For example, turkeys passively immunized with antibodies to E. coli IROMPS have been shown to be partially protected against E. coli septicemia. [See, C.A. Bolin et al, Infect. Immun., 55:1239-1242 (1987); see, also, J.R. Black et al, Infect. Immun., 54:710-713 (1986); and M. Fohn et al, Infect. Immun., 55:3065-3069 (1987)].

H. Deneer et al, cited above, have reported the increased synthesis in vitro of two proteins, one being 105K and the other 75K, when virulent A. pleuropneumoniae serotype 1 was grown under iron-restricted conditions. The function of these proteins has not been specifically identified; however, it is stated that one or both of these protein may serve as a receptor for siderophore-iron complexes.

The prophylaxsis of A. pleuropneumoniae infection presently relies on vaccines which consist of bacterins containing whole cells. Inzana et. al., Inf. Immun., 56 (8) 1880-89, 1988 discloses that protection against lethal infection by H. pleuropneumoniae was only induced when animals were immunised with live encapsulated or non-encapsulated bacteria. This type of vaccine has been unsuccessful in controlling and preventing this disease. The whole-cell bacterins do not prevent chronic forms of the disease, nor are the bacterins capable of halting or preventing development of the subclinical carrier state. Additionally they do not induce immunity to heterologous serotypes of A. pleuropneumoniae [R. Higgins et al, Can. Vet. J., 26:86-89 (1985); R. Nielsen et al, Acta Vet. Scand., 27:453-455 (1987)].

There remains a need in the art of veterinary practice for vaccines effective against infection of animals by A. pleuropneumoniae.

### Summary of the Invention

The present invention provides novel vaccine components and compositions and a method of enhancing the immune response to known vaccines.

The present invention provides novel vaccine compositions for protecting swine from infection by Actinobacillus pleuropneumoniae comprising an effective amount of an iron repressible outer membrane protein (IROMP) which is useful as a protective antigen. In a preferred embodiment this IROMP protein is isolated from A. pleuropneumoniae, serotype 5, and characterized by a molecular weight of approximately 105 kD.

The vaccines of the present invention contain an A. pleuropneumoniae capsular extract, as well as the IROMP.

The present invention further provides a method for enhancing the immune response of an animal to any selected vaccine composition against a pathogen which is characterized by the presence of an IROMP by incorporating an immunogenic amount of an appropriate immunopotentiating iron repressible outer membrane protein into the vaccine composition. The vaccine is thereby provided with increased antigenic and immunogenic properties.

Other aspects and advantages of the present invention are described further in the following detailed description of preferred embodiments thereof.

### Detailed Description of the Invention

The present invention provides vaccine compositions for the prophylaxis of diseases resulting from infections of pathogenic microorganisms, which vaccines contain an iron repressible outer membrane protein (IROMP) which is characteristic of the selected microorganism.

According to the present invention, there is provided a vaccine composition for protecting swine from infection by Actinobacillus pleuropneumoniae comprising:
(a) one or more iron repressible outer membrane proteins (IROMPs) obtained from a first serotype of an Actinobacillus pleuropneumoniae strain, said strain being cultured under iron-restricted conditions in the presence of an iron chelator, wherein said strain is characterised by being capable of generating an IROMP when cultured under iron-restricted conditions in the presence of an iron chelator, whereby said strain generates said IROMPs; or
   a capsular extract of said iron-restricted culture of said first serotype of said Actinobacillus pleuropneumoniae strain containing, said IROMPs;
(b) one or more capsular extracts from cultures comprising one or more serotypes of one or more Actinobacillus pleuropneumoniae strains which may be the same as or different from said first serotypes, said one or more serotypes not being cultured under iron-restricted conditions in the presence of an iron chelator; and
(c) a pharmaceutically acceptable carrier.

The vaccine composition of this invention is useful in the prophylaxsis of disease caused by infection of swine by Actinobacillus (Haemophilus) pleuropneumoniae, which causes an acute pneumonia with fibrinous pleuritis or chronic lung lesions. While the embodiment described herein in detail employs A. pleuropneumoniae as the pathogen, it is expected that the teachings of this invention may be easily adapted to vaccine compositions for other pathogens by one of skill in the art. The teachings of the present invention are not limited to the exemplified vaccine compositions alone.

The A. pleuropneumoniae IROMP has been characterized as having a molecular weight of approximately 105 kiloDaltons (kD) [see, e.g., H. Deneer, et al., Infect. Immun., 57:798-804 (1989)]. The inventors have surprisingly found that when the A. pleuropneumoniae IROMP is incorporated into vaccine compositions containing other antigens of that pathogen, e.g., whole cell A. pleuropneumoniae bacterin or A. pleuropneumoniae capsular extract antigens, the vaccines show marked improvement in protection against challenge with homologous A. pleuropneumoniae serotypes, and significant improvement in cross-protection against heterologous serotypes (e.g. serotype 1).

The embodiments described herein in detail employ the IROMP from A. pleuropneumoniae, serotype 5. However, it is expected that IROMPs of other A. pleuropneumoniae serotypes and other species pathogens may also be useful in either A. pleuropneumoniae vaccines or other pathogen vaccines, respectively, by following the teachings of this invention. The present invention is not limited to the exemplified IROMP only. IROMPS from a selected microorganism may similarly be used with antigens other than those described above. Additionally, the IROMP from one microorganism is expected to be useful in a vaccine containing antigens from a different microorganism.

An IROMP useful in the present vaccine compositions may be isolated and identified in a culture of the selected pathogen. Briefly described, an IROMP is isolated by culturing the selected microorganism in a suitable medium, such as PPLO broth or the media formulations identified specifically in Example 1 below. Because an IROMP is made only under iron restricted conditions, a selected iron chelator, such as 2-2 dipyridyl, is added to the culture. The presence of the IROMP is thereafter detected in the culture by the addition of a selected dye, such as Congo Red, and the performance of a dye-binding assay on a sample of the culture. This methodology is described in detail in Example 1 below.

Once the IROMP is identified as present in the culture, untreated portions of the culture may then be used for the preparation of the vaccine formulation, comprising capsular extract antigen preparations. Alternatively, other isolation techniques, such as that described by Deneer et al, cited above, may be employed, and/or the IROMP itself may be isolated from the microorganism and obtained by chemical synthesis or recombinant engineering techniques. Additionally, it is contemplated that an IROMP protein which has been isolated from culture can be added as a separate component, for example, in an amount from about 0.1 to 1.8 mL, to a vaccine composition containing another antigen.

A. pleuropneumoniae vaccines of the invention may be prepared as pharmaceutical compositions containing an effective amount of the 105 kD IROMP as an active ingredient. If the IROMP is isolated from the culture, it may be added in effective amounts to other vaccine formulations in a suitable pharmaceutical carrier.

In a preferred embodiment, the 105 kD IROMP can be a component in an immunostimulatory preparation, with an A. pleuropneumoniae whole cell vaccines. A novel vaccine comprising a whole cell A. pleuropneumoniae bacterin and an A. pleuropneumoniae 105 kD IROMP may be prepared as follows. A selected A. pleuropneumoniae strain which has been identified as possessing the ability to generate an IROMP under iron-restricted conditions may be conventionally cultured in suitable media in the presence of an iron chelator and inactivated with a conventional inactivating agent, such as formalin (formaldehyde solution USP). The inactivating agent may be added in an amount between approximately 0.2% to 1.0% by weight. Preferably the inactivating agent is added to the culture at a final concentration of 0.3%. Other known inactivating agents may also be used, such as, glutaraldehyde or β-propiolactone (BPL). When utilized as inactivating agents, glutaraldehyde is preferably added in a concentration of between 0.05% to 0.5% by weight and BPL is preferably added in a concentration of between 0.1% to 0.3% by weight. However, one of skill in the art can readily determine the concentration and time parameters necessary for inactivation with a selected inactivating agent.

Once the inactivating agent is added, the culture is incubated for a suitable period, e.g., about 18 to 48 hours at about 37°C. The inactivating agent may be removed or neutralized by conventional means, and the culture incubated again to complete neutralization of the inactivating agent, e.g. with sodium bisulfate (37%) for up to 24 hours at 20-25°C.

Once inactivated, the culture containing the IROMP may then be formulated into a suitable vaccine.

The 105 kD IROMP can be administered in an immunostimulatory preparation, such as an A. pleuropneumoniae subunits vaccine. One embodiment of this aspect of the invention is a novel vaccine comprising A. pleuropneumoniae capsular extract proteins and an A. pleuropneumoniae 105 kD IROMP. Capsular extract is prepared by culturing A. pleuropneumoniae, incubating, chilling the culture, obtaining a bacterial cell pellet which is then suspended in PBS, heating the bacterial cells, centrifuging again, collecting the supernatant and sterilizing the extracted product. Conditions for this method are detailed in Example 2.

The IROMP may or may not be present in the immunostimulatory preparation depending upon the presence or absence of chelator in the bacterial culture from which the capsular extracts are prepared. However, according to this invention, the presence of IROMP is preferred. It is also preferred for such a vaccine that capsular extract proteins be prepared from more than one strain of the bacteria. The protein present in the culture media is concentrated, and the protein concentration with IROMP and the total carbohydrate concentration are calculated, each by known methods. Calculations are performed to determine the volume of extract antigen with IROMP necessary to prepare a preferred dosage of a capsular extracts vaccine.

An inactivating agent, e.g., 25% glutaraldehyde, is then employed to couple the extract antigens from different serotypes (e.g. App 1, 5 and 7), thus cross-linking the protein components which results in the formation of a macromolecule. The mixture of extract antigen with IROMP and glutaraldehyde is then incubated, preferably at 20-25°C for between approximately 30 minutes to 180 minutes. To neutralize excess glutaraldehyde, a lysine stock solution is added to this combination and that mixture incubated.

This formulation may then be emulsified for administration by mixture with a cell free supernatant fraction from the capsular extract vaccine preparation, described below. This fraction may be used as a diluent because it may contain small amounts of IROMP. Other additives to this formulation may include a selected adjuvant, and conventional vaccine preservatives, detergents and emulsifiers, such as e.g. merthiolate, Tween 80, and Span 80. A detailed description of such a vaccine is described in detail in Example 3, part B, below.

Still another preferred vaccine embodiment of this invention is a trivalent vaccine, containing an IROMP, preferably from A. pleuropneumoniae serotype 5, and capsular extracts from one or more other strains of the same microorganism. The additional strains do not provide IROMPS. Example 3, part C, is a detailed description of one such vaccine. Preferably the trivalent vaccine employs an IROMP from one strain of microorganism, and a capsular extract from at least a second and third strain. Additional capsular extracts from additional strains may also be employed. This provides the vaccine with the ability to confer protection against all three strains of the microorganism, e.g., A. pleuropneumoniae.

The IROMP of the present invention is also expected to be useful in vaccines containing more than two other antigens. Other antigens which are A. pleuropneumoniae antigens may also be present in vaccines of this invention, for example, hemolysins and cytotoxins, may also be used in conventional dosages in these vaccines to provide additional immunogenicity.

It is preferred that the vaccines of the invention, when in pharmaceutical preparation, be present in unit dosage forms. For purposes of this invention, an immunogenic amount of the 105 kD IROMP, is a 0.28 optical density 488 nm (O.D. 488 nm) reduction per milliliter of bulk fluids or one tenth to nine tenths of a single dose of vaccine. A whole cell bacterin with IROMP vaccine preferably contains between 1 x 10⁸ to 1 x 10⁹ colony forming units (CFU), more preferably 5 x 10⁸ CFU. Preferably, in a vaccine containing a capsular extract, there is at least between 25-50 µg/serotype of capsular extract per dose of vaccine. A capsular extract with IROMP vaccine also preferably contains between 25-50 µg total carbohydrates per dose.

The appropriate therapeutically effective dose can be determined readily by those of skill in the art based on the above immunogenic amounts. Accordingly, a pharmaceutical preparation provides a unit dosage of between 0.1 to 1.8 mLs of a sterile solution of an immunogenic amount of the active ingredients. One embodiment of the invention includes a vaccine dosage unit comprising 0.1 to 1.8 mL of a sterile solution of an immunogenic amount of a A. pleuropneumoniae 105 kD IROMP with a whole cell A. pleuropneumoniae bacterin. Another embodiment includes a vaccine dosage unit of 0.1 to 1.8 mL of a sterile solution of an immunogenic amount of a A. pleuropneumoniae 105 kD IROMP with A. pleuropneumoniae capsular proteins. Still a further embodiment is a dosage unit comprising 0.1 to 1.8 mL of a sterile solution of an immunogenic amount of one of above two vaccine embodiments and one or more additional antigenic components.

A desirable dosage regimen involves administration of two doses of desired vaccine composition. The mode of administration of the vaccines may be any suitable route which delivers the vaccine to the host. However, the vaccine is preferably administered by intramuscular injection. Other modes of administration may also be employed, where desired, such as orally, intranasally, intradermally, or intraperitoneally.

Present investigations with swine employ intramuscular injection of two doses of vaccine administered to the subject animal three weeks apart. It will be understood, however, that the specific dose level for any particular animal will depend upon a variety of factors including the age, general health, sex, and diet of the animal; the species of the animal; the time of administration; the route of administration; interactions with any other drugs being administered; and the degree of protection being sought. Of course, the administration can be repeated at other suitable intervals if necessary or desirable. Thus the dosage amounts, mode and manner of administration of these vaccines will be determined by the veterinarian.

Vaccines of this invention provide increased protection against challenge with virulent organisms of A. pleuropneumoniae serotype 5. These vaccines can induce in an animal a superior immune response to A. pleuropneumoniae and can provide additionally cross protection between heterologous serotypes, thus mimicking the immune status of an animal surviving a natural infection. The specific mechanism of protection induced by the vaccine compositions of the present invention is the increased protection (lower mean lung lesion score) when compared to vaccines without 105 kd IROMP protein as indicated by the in vivo animal tests described in Example 4, Table 3.

The examples which follow illustrate preferred methods for isolating the A. pleuropneumoniae 105 kD IROMP and for preparing and testing a variety of vaccines containing this novel component. These examples are illustrative only and do not limit the scope of the present invention.

### EXAMPLE 1 - ISOLATION OF THE A. PLEUROPNEUMONIAE 105 kD PROTEIN

A. pleuropneumoniae serotype 1 (strain Schelkopf) and serotype 5 (strain K-17) [both available from SmithKline Beecham Animal Health, Norden Laboratories, Lincoln, Nebraska] were initially tested for their potential to secrete an IROMP in a defined synthetic medium (MI_{E}), Bitek [Difco Laboratories, Detroit, Mich.] and PPLO [Difco] broth upon addition of the iron chelator 2-2' Diyridyl [Sigma Chemical Co., St. Louis, Mo.] to a final concentration of 200 µM, as described in detail below.

MI_{E} medium (20 liters) is prepared by combining a mixture of the three stock solutions described below, adjusting the combined medium to a neutral pH, and filter sterilizing the combined medium.

Stock Solution #1 is prepared (17399.3 mL) by dissolving the following ingredients in order, while stirring: 1 N HCl 280 mL; L-Cysteine 4.0 g; Tyrosine 4.0 g; Distilled Water 16319.3 mL; Leucine 6.0 g; Arginine 6.0 g; Glycine 0.6 g; Lysine 1.0 g; Methionine 2.0 g; Serine 2.0 g; Uracil 2.0 g; 1 N NaOH 800 mL; Hypoxanthine 0.4 g; Inosine 40.0 g; K₂HPO₄ 69.6 g; KH₂PO₄ 54.4 g; and Yeast Extract 120.0 g.

Stock Solution #2 (2400.7 mL) is prepared by dissolving the following ingredients in order, with stirring: Distilled water 2290 mL; Aspartic Acid 10.0 g; Glutamic Acid 26.0 g; Sodium Chloride 116.0 g; Potassium Sulfate 20.0 g; Magnesium Chloride (6H₂O) 8.0 g; Calcium Chloride (Anhydrous) 0.444 g; EDTA 0.07 g; Ammonium Chloride 4.4 g; 10 N NaOH 23.6 mL. The following ingredients are then dissolved with stirring: Tween 80 0.4 mL; DL-Lactic Acid (60% Solution) 26.6 mL; and Glycerol 60.0 mL.

Stock Solution #3 (200 mL) is prepared by dissolving 20.0 g soluble starch in 200 mL distilled water with gentle heating and stirring.

Stock Solution #2 is added to Stock Solution #1. Stock Solution #3 is then added to the mixture. The pH of the assembled medium is adjusted to 7.3 ± 0.1 pH units and the complete medium is filter-sterilized. This solution may be stored at approximately 4°C for up to two weeks.

Prior to use of the MI_{E} broth, 20 mL of sterile NAD stock solution is added. This solution is prepared by dissolving, in order, 1.0 g Nicotinamide Adenine Dinucleotide (NAD), 1.0 g Thiamine, 1.0 g Calcium Pantothenate, and 100 mL of distilled water, while stirring. The medium is again filter sterilized and stored at -20°C.

PPLO Broth (approximately 1 liter) is prepared by dissolving, in order, 21.0 g PPLO Broth w/o CV [Difco], 5.0 g Glucose, and 1000 mL distilled water with stirring. The medium is adjusted to a pH of approximately 7.3 and filer sterilized. Prior to use, 1.0 mL of a sterile NAD stock solution is added and 100 mL of sterile distilled water containing 10.0 mL of sterile Tween 80 is added.

BiTek Broth (approximately 1 liter) is prepared by dissolving, with stirring, 29.2 g BiTek Broth [Difco] in 1000 mL distilled water. The medium is adjusted to a pH of approximately 7.3 and filter sterilized. Prior to use, 1.0 mL of a sterile NAD stock solution is added.

All three of the above described media were tested for their potential to produce the IROMP. Results, described in more detail below suggested PPLO broth was best suited for production of IROMP.

The presence of 105 kD protein was determined by a dyebinding (Congo Red) assay, performed following the method described by Deneer and Potter, cited above.

### A. Serotype I

A. pleuropneumoniae serotype 1 was inoculated directly from the frozen master seed, 1%, into 100 ml of PPLO broth supplemented with 0.5% glucose and 0.1% NAD [Sigma] solution. The culture was incubated overnight at 37°C. Thereafter, 500 ml of PPLO broth supplemented with 0.5% glucose and 0.1% NAD solution was inoculated with a 2% seed from this overnight culture of A. pleuropneumoniae serotype 1 and incubated at 37°C.

Stock solution of 2-2' dipyridil (final concentration of 200 µM) was added to the 500 ml culture of A. pleuropneumoniae serotype 1 when the optical density reached 1.0 at 650 nm. After a 90 minute incubation at 37°C, the cells were harvested by centrifugation (8000 x g, 10 minutes). The cell pellet was resuspended in 100 ml of sterile phosphate buffered saline (PBS), centrifuged at 8000 rpm for 15 minutes, and resuspended in PBS to an OD₆₅₀ₙₘ = 1.0.

One ml of Congo Red per 100 ml of A. pleuropneumoniae culture was added and mixed. A sample was immediately taken, 1.0 ml into each of 4 microfuge tubes, and spun for one minute in a microfuge and the supernatant read at 488 nm (blanked with PBS). PPLO broth with 0.1% NAD was used as a control. The culture was maintained at 37°C with agitation. A sample (4.0 ml) was harvested at 0, 20, 40 and 60 minutes post-addition of Congo Red.

The results of this assay illustrated in Table 1A below show the effects of iron restriction upon the outer membrane proteins of A. pleuropneumonia serotype 1. The uptake of Congo Red is expressed as the depletion of Congo Red from the solution.

**TABLE 1A**

| Time (minutes) | OD at 488 nm | |
|---|---|---|
| | chelator | control |
| 0 | 0.34 | 0.32 |
| 20 | 0.14 | 0.27 |
| 40 | 0.10 | 0.20 |
| 60 | 0.06 | 0.18 |

Thus, A. pleuropneumoniae serotype I is capable of binding Congo Red under iron-restricted conditions. These results demonstrate that the IROMP preparation has the ability to bind Congo Red and further suggest that IROMP plays a role in the acquisition of complex iron such as hemin during in vivo growth.

### B. Serotype 5

A. pleuropneumoniae serotype 5 was grown in 14 liter fermentors containing 10 liters of PPLO broth supplemented with 0.5% glucose, 0.02% Tween 80 and NAD (final concentration of 10 µg/ml). The broth was inoculated with a 2% seed inoculum of A. pleuropneumoniae serotype 5. When the culture had an OD₆₅₀ₙₘ = 1.0 (four hours after inoculation), chelator was added to a final concentration of 200 µM.

Ninety minutes later, 5 liters of culture was taken for capsular antigen preparation (see Example 2). Of this, 200 ml was taken for processing in the Congo Red binding assay. The 200 ml sample was centrifuged and the cell pellet washed twice with PBS. The cells were resuspended in sterile PBS to an OD₆₅₀ₙₘ of 1.4. Sterile Congo Red solution was added to a final concentration of 30 µg/ml and the assay performed as described above. The sample indicated an OD₄₈₈ₙₘ reduction of 0.075 units per ml of fermentor culture. The results of this assay illustrated in Table 1B below show the effects of iron restriction upon the outer membrane proteins of A. pleuropneumonia serotype 5.

**TABLE 1B**

| Time (minutes) | OD at 488 nm chelator |
|---|---|
| 0 | 0.27 |
| 10 | 0.19 |
| 20 | 0.11 |
| 30 | 0.07 |
| 40 | 0.05 |
| 50 | 0.04 |
| 60 | 0.03 |

This illustrates the ability of A. pleuropneumonia serotype 5 to bind Congo Red. These results demonstrate that the IROMP preparation has the ability to bind Congo Red and suggest that IROMP may play a role in the acquisition of complex iron, such as hemin, during in vivo growth.

The remaining 5 liters of culture not employed in capsular antigen preparation nor used in the Congo Red assay were inactivated with formalin which was added to a final concentration of 0.3% for preparing whole cell inactivated vaccine with IROMP, described in detail in Example 3.

### EXAMPLE 2 - PREPARATION OF CAPSULAR EXTRACT ANTIGENS

A bacterial capsular extract antigen is generally prepared as follows. MI_{E} broth is inoculated with a 1% seed of A. pleuropneumoniae which was previously frozen at -70°C in standard bacterial drying menstrum medium. The culture is then incubated at 37°C with agitation for 16-17 hours. A 14 liter fermenter containing 10 liters of MI_{E} broth is inoculated with a 1 to 5 seed from a 16-17 hour culture of A. pleuropneumoniae. Fermentation parameters for the 14 liter fermenter are as follow: temperature is 37°C ± 1°C, pH 7.0 - 7.3 and is controlled with 5N NaOH, D.O. is maintained at 30% with sterile house air, with agitation at between 100 to 250 rpm. The culture is then allowed to grow until it reaches mid to early late log growth phase (4 to 16 hours) as determined by optical density at 650ₙₘ. After the culture has reached mid to early late log growth, the culture is chilled to 20°C or lower and centrifuged, or subjected to microfiltration. The bacterial cell pellet is resuspended in PBS (one-tenth to one-thousandth of the original culture volume) with agitation for 1 to 10 hours at 4°C. After the bacterial cells are thoroughly resuspended the suspension is heated to 56°C ± 1°C for 60 minutes ± 30 minutes. The material is then centrifuged or is subjected to microfiltration and the supernatant collected. Sterile 10% merthiolate and 10% Ethylene-diamine tetra acetic acid (EDTA, disodium or tetra sodium salt) are added as preservatives. Final concentration shall not exceed 0.01% and 0.07% (weight per volume), respectively. The extracted product is sterile filtered through a 0.45 and 0.2 µm filter, into a sterile storage vessel and stored at 2°C-7°C until assembled. If needed, the product is first clarified by filtration or centrifugation and then filter sterilized.

Since no iron chelator was added to the culture medium, the above-described procedure produces capsular extract antigen with no IROMP. These capsular extract antigens may be used as controls. Alternatively, the procedure may be followed to provide additional antigens from bacterial strains incapable of producing IROMPs.

However a capsular extract with IROMP can be made by following the above procedure, modified by adding a selected iron chelator, such as 2-2' dipyridyl, to the growth media to generate the IROMP.

### Trivalent vaccines

Trivalent vaccines, i.e., vaccines containing the 105 kD IROMP protein isolated from A. pleuropneumoniae serotype 5, serotype 1 capsular extract, and serotype 7 capsular extract were tested for efficacy. Serotype 5 was prepared as described above in Example 1. Serotype 1 was grown in MI_{E} medium as described above in Example 1. Serotype 7 [SmithKline Beecham Animal Health, Norden Laboratories, Lincoln, Nebraska] was grown as described above for serotype 1.

500 Doses of trivalent capsular extract bacterin with IROMP at 25 µg of carbohydrate per dose were prepared as follows. First, the protein concentration of the bulk lot of each A. pleuropneumoniae serotype (1, 5 + 105 kd, and 7) capsular extract antigen was determined by the Lowry Method of Protein determination [Herbert, Phipps and Strange, cited above, p. 249-252]. Protein concentration was 1,395 µg/mL for serotype 1, 1,000 µg/mL for serotype 5 with 105 kD protein, and 1,475 µg/mL for serotype 7.

The total carbohydrate concentration of the bulk lot of A. pleuropneumoniae serotype (1, 5 + 105 kd, and 7) capsular extract antigen, as determined by the Phenol Method of total carbohydrate determination [Herbert, Phipps and Strange, cited above, p. 272-277], was found to be 1,561 µg/mL for serotype 1, 600 µg/mL for serotype 5 with 105 kD IROMP, and 1250 µg/mL for serotype 7.

The number of milliliters of each A. pleuropneumoniae capsular extracted antigen that is needed to prepare 500 doses of bacterin containing 25 µg of total carbohydrate per serotype per dose is determined as demonstrated in the following exemplary equations.

| Serotype | Total Carbohydrate Concentration | Total Volume |
|---|---|---|
| 1 | 25 µg/dose/1561 µg/mL x 500 doses = | 8.0 mL |
| 5 + 105 kD | 25 µg/dose/600 µg/mL x 500 doses = | 21.0 mL |
| 7 | 25 µg/dose/1250 µg/mL x 500 doses = | 10.0 mL |

The 8.0 mL of serotype 1, 21.0 mL of serotype 5 with 105 kD protein, and 10.0 mL of serotype 7 capsular extracted antigens are then coupled with 25% glutaraldehyde. 1.0 mL of 25% glutaraldehyde is added per gram of protein present in the 500 doses of bacterin.

To determine the amount of 25% glutaraldehyde required to couple the trivalent capsular extracted antigens (total volume = 39.0 mL) the following calculation is made:

| Protein Bulk Material | Vol. Required Concentration | | Total Protein for 250 doses | Concentration |
|---|---|---|---|---|
| Serotype 1 | 1395 µg/mL | X | 8.0 mL = | 11,160 µg |
| Serotype 5 + 105 kD | 1000 µg/mL | X | 21.0 mL = | 21,000 µg |
| Serotype 7 | 1475 µg/mL | X | 10.0 mL = | 14,750 µg |
| | | Total protein = | | 46,910 µg |

Therefore, 0.047 mL of 25% glutaraldehyde is required for coupling.

8.0 mL of serotype 1 capsular extract preparation, 21.0 mL of serotype 5 capsular extract preparation containing the 105 kD protein, and 10.0 mL of serotype 7 capsular extract preparations are combined. 0.047 mL of 25% glutaraldehyde is added to the above preparation. The mixture is then incubated at room temperature for 1 hour with agitation. After the one hour incubation, 0.047 mL of Lysine stock solution (12.5 mg/mL) is added. The mixture is incubated at room temperature for two hours with agitation, and then stored at 4°C.

The trivalent bacterin containing 25 µg/serotype/dose is then emulsified as follows: 39.0 mL of A. pleuropneumoniae serotype 1, 5 and 7 glutaraldehyde coupled capsular extracted antigen (described above) (0.088 mL volume/dose) is mixed together with 1.00 mL 10% Merthiolate-EDTA (0.002 mL vol/dose), 0.6 mL 10% EDTA (0.0012 mL vol/dose), and 889.4 mL of Supernatant of A. pleuropneumoniae serotype 5 with chelator (1.7788 mL vol/dose) obtained prior to heat extraction. 14.00 mL Tween 80 (0.028 mL vol/dose) and 6.00 mL Span 80 (0.012 mL vol/dose) are added to 50.00 mL Amphigen (0.10 mL vol/dose) to form the oil fraction. The oil fraction is then added to the glutaraldehyde coupled preparation, 10% Merthiolate-EDTA, 10% EDTA and supernatant fraction while the emulsifier is turned on. The bacterin is then emulsified for 2 minutes.

### EXAMPLE 4 - VACCINATION EXPERIMENTS

Each vaccine type, whole cell bacterin and capsular extract vaccine, containing the 105 kD protein was tested in pigs for protective capabilities by a vaccination-challenge experiment. Similar vaccines were prepared as described above except the bulk fluids did not contain the 105 kD protein. Appropriate controls, non-vaccinated pigs, were similarly tested.

### A. Monovalent vaccines

Using whole cell bacterins and capsular extract vaccines prepared as described in Example 3, Parts A and B, with and without the 105 kD protein isolated from A. pleuropneumoniae serotype 5, 2 mL vaccination dosages were administered intramuscularly into the side of the neck of pigs from Eberle farms. The vaccines are:
Vaccine XHPP-1: Whole cell bacterin containing no 105 kD protein was administered at 5 x 10⁸ CFU/dose.
Vaccine XHPP-2: Whole cell bacterin containing the 105 kD protein and described above in Example 2 was administered at 5 x 10⁸ CFU/dose.
Vaccine XHPC-16: Capsular extract vaccine containing no 105 kD protein was administered at 50 µg carbohydrate/dose.
Vaccine XHPC-18: Capsular extract vaccine containing 105 kD protein was administered at 50 µg carbohydrate/dose.

First vaccination was when pigs were at 3-4 weeks of age. A second vaccination was administered 3 weeks after the first inoculation.

One week after the second vaccination, challenge with the appropriate serotype, A. pleuropneumoniae 5, was done by intranasal instillation (0.5 ml per nostril) of a virulent culture. The cell count in the challenge inoculum was 1.19 x 10⁹ CFU/ml. One week after challenge the animals were necropsied. Animals which succumbed to challenge were necropsied soon after death.

The results of these vaccination experiments are illustrated in Table 2 below.

**TABLE 2**

| Vaccine | No. Pigs | Mean % Lung Damage | Percent reduction in Lung involvement |
|---|---|---|---|
| XHPP-1 | 10 | 32.24 | 37.56 |
| XHPP-2 | 10 | 19.18 | 62.85 |
| XHPC-16 | 10 | 42.38 | 17.92 |
| XHPC-18 | 10 | 10.31 | 80.03 |
| None | 10 | 51.63 | - |

The results show a significant decrease in mean lung damage and significant increase in the percent reduction in lung involvement for the groups vaccinated with the vaccine compositions containing the 105 kD IROMP (XHPP-2 and XHPP-18).

### B. Trivalent vaccines

Using the trivalent vaccines containing the 105 kD protein isolated from A. pleuropneumoniae serotype 5, with two strains of capsular extract prepared as described in Example 3, Part C, and vaccines without the IROMP, 2 mL vaccination dosages were administered intramuscularly into the side of the neck of pigs from Eberle farms.

The vaccines employed are:
Vaccine XHPC-19: A capsular extract vaccine containing 25 µg of total carbohydrate/ serotype/dose and no 105 kD protein.
Vaccine XHPC-21: A capsular extract vaccine containing 105 kD protein and 25 µg of total carbohydrate/serotype/dose.
Vaccine XHPC-20: A capsular extract vaccine containing 50 µg of total carbohydrate/serotype/ dose and no 105 kD protein.
Vaccine XHPC-22: A capsular extract vaccine containing the 105 kD protein and 50 µg of total carbohydrate/serotype/dose.

Three cultures, serotype 1, serotype 5, and serotype 7 were used to challenge each animal in a vaccine group and control animal group. These cultures contained the following cell counts:
A. pleuropneumoniae serotype 1 = 2.19 x 10⁹ CFU/ml,
A. pleuropneumoniae serotype 5 = 8.85 x 10⁸ CFU/ml, and
A. pleuropneumoniae serotype 7 = 1.01 x 10⁹ CFU/ml.

Pigs were challenged as described above for the monovalent vaccinations in Part A. The controls consisted of a group of pigs which received a placebo (PBS adjuvanted amphigen) vaccine. The placebo was administered on the same vaccine schedule (same dose and route) as the pigs that received either XHPC-19, XHPC-20, XHPC-21, or XHPC-22. The results are reported below in Table 3.

**TABLE 3**

| Vaccine | No. Pigs | Challenge Serotype | Mean % Lung Damage | Percent reduction in Lung involvement |
|---|---|---|---|---|
| XHPC-19 | 19 | 1 | 12.27 | 73.21 |
| | 18 | 5 | 30.01 | 55.10 |
| | 20 | 7 | 20.20 | 59.60 |
| XHPC-21 | 10 | 1 | 4.0 | 91.27 |
| | 10 | 5 | 7.89 | 88.20 |
| | 10 | 7 | 20.25 | 59.54 |
| XHPC-20 | 19 | 1 | 26.43 | 42.30 |
| | 20 | 5 | 25.80 | 61.40 |
| | 20 | 7 | 21.08 | 57.88 |
| XHPC-22 | 10 | 1 | 8.96 | 80.44 |
| | 10 | 5 | 35.51 | 46.88 |
| | 10 | 7 | 17.93 | 64.18 |
| None | 9 | 1 | 45.80 | - |
| | 10 | 5 | 66.85 | - |
| | 10 | 7 | 50.05 | - |

These results demonstrate that animals which were vaccinated with vaccines that contained the 105 kd protein (XHPP-21 and XHPP-22) had a greater reduction in percent lung damage in most cases compared to animals which received vaccine without the 105 kd protein.

The trivalent vaccine is particularly advantageous, as compared to the monovalent vaccine, because the trivalent vaccine offers protection against the three serotypes contained in the vaccine. In contrast, the monovalent vaccine offers protection only against the single serotype contained in its formulation.

Numerous modifications and variations of the present invention are included in the above-identified specification and are expected to be obvious to one of skill in the art. Other conventional adjuvants and inactive vaccine components may be employed in the formulations and selected by one of skill in the art. The dosages and administration protocols for use of these vaccine compositions may also be adjusted by one of skill in the art based on the animal to be vaccinated, the disease for which protection is desired and other related factors. Such modification and alterations are believed to be encompassed in the scope of the claims appended hereto.

## Claims

1. A vaccine composition for protecting swine from infection by *Actinobacillus pleuropneumoniae* comprising:
a) one or more iron repressible outer membrane proteins (IROMPs) obtained from a first serotype of an *Actinobacillus pleuropneumoniae* strain, said strain being cultured under iron-restricted conditions in the presence of an iron chelator, wherein said strain is characterised by being capable of generating an IROMP when cultured under iron-restricted conditions in the presence of an iron chelator, whereby said strain generates said IROMPs; or
a capsular extract of said iron-restricted culture of said first serotype of said *Actinobacillus pleuropneumoniae* strain containing said IROMPs;
b) one or more capsular extracts from cultures comprising one or more serotypes of one or more *Actinobacillus pleuropneumoniae* strains which may be the same as or different from said first serotype, said one or more serotypes not being cultured under iron-restricted conditions in the presence of an iron chelator; and
c) a pharmaceutically acceptable carrier.

2. A vaccine composition according to claim 1, wherein said one or more capsular extracts from cultures comprising one or more serotypes is prepared from *A.pleuropneumoniae* serotypes 1, 5 and 7, or from serotypes 1 and 7, or from serotypes 1 and 5.

3. A vaccine composition according to claim 1 or 2, wherein said capsular extract from said first serotype is prepared from *A.pleuropneumoniae* serotype 5.

4. A vaccine composition according to claim 1, 2 or 3, wherein said iron chelator is 2,2-dipyridyl.

5. Use of a vaccine composition according to any preceding claim in the manufacture of a medicament for use in vaccination of swine against infection by *A.pleuropneumoniae*.

6. Use according to claim 5 wherein said medicament is to be administered in an immunostimulatorily effective amount of said composition.

## Patentansprüche

1. Impfstoff-Zusammensetzung, um Schweine vor Infektion durch Actinobacillus pleuropneumoniae zu schützen, umfassend:
a) ein oder mehrere Eisen-reprimierbare äußere Membranproteine (IROMP), erhalten aus einem ersten Serotyp eines Actinobacillus pleuropneumoniae-Stamms, wobei der Stamm unter eisenarmen Bedingungen in Anwesenheit eines Eisen-Chelators gezüchtet wird, wobei der Stamm dadurch gekennzeichnet ist, daß er ein IROMP erzeugen kann, wenn er unter eisenarmen Bedingungen in Anwesenheit eines Eisen-Chelators gezüchtet wird, wodurch der Stamm die IROMP erzeugt; oder
einen Kapselextrakt der eisenarmen Kultur des ersten Serotyps des Actinobacillus pleuropneumoniae-Stamms, der diese IROMP enthält;
b) ein oder mehrere Kapselextrakte aus Kulturen, die ein oder mehrere Serotypen von einem oder mehreren Actinobacillus pleuropneumoniae-Stämmen umfassen, die gleich dem ersten Serotyp oder davon verschieden sein können, wobei die einen oder mehreren Serotypen nicht unter eisenarmen Bedingungen in Anwesenheit eines Eisen-Chelators gezüchtet wurden; und
c) einen pharmazeutisch verträglichen Träger.

2. Impfstoff-Zusammensetzung nach Anspruch 1, wobei der eine oder die mehreren Kapselextrakte aus Kulturen, die einen oder mehrere Serotypen umfassen, aus A. pleuropneumoniae-Serotypen 1, 5 und 7 oder aus Serotypen 1 und 7 oder aus Serotypen 1 und 5 hergestellt sind.

3. Impfstoff-Zusammensetzung nach Anspruch 1 oder 2, wobei der Kapselextrakt aus dem ersten Serotyp aus A. pleuropneumoniae-Serotyp 5 hergestellt ist.

4. Impfstoff-Zusammensetzung nach Anspruch 1, 2 oder 3, wobei der Eisenchelator 2,2-Dipyridyl ist.

5. Verwendung einer Impfstoff-Zusammensetzung nach einem vorangehenden Anspruch bei der Herstellung eines Arzneimittels zur Verwendung bei der Impfung von Schweinen gegen Infektion durch A. pleuropneumoniae.

6. Verwendung nach Anspruch 5, wobei das Arzneimittel in einer immunostimulatorisch wirksamen Menge der Zusammensetzung zu verabreichen ist.

## Revendications

1. Composition de vaccin destinée à protéger les porcs contre une infection par Actinobacillus pleuropneumoniae, comprenant :
a) une ou plusieurs protéines de membrane extérieure aptes à la répression par le fer (IROMP) obtenues à partir d'un premier sérotype d'une souche d'Actinobacillus pleuropneumoniae, ladite souche étant cultivée dans des conditions de limitation en fer en présence d'un chélateur de fer, ladite souche étant caractérisée par la capacité à engendrer une IROMP lors de sa culture dans des conditions de limitation en fer en présence d'un chélateur de fer, ladite souche engendrant ainsi lesdites IROMP ; ou
un extrait capsulaire de ladite culture, obtenu dans des conditions de limitation en fer, dudit premier sérotype de ladite souche d'Actinobacillus pleuropneumoniae contenant lesdites IROMP ;
b) un ou plusieurs extraits capsulaires de cultures comprenant un ou plusieurs sérotypes d'une ou plusieurs souches d'Actinobacillus pleuropneumoniae qui peuvent avoir un sérotype identique audit, ou différent dudit, premier sérotype, ce ou ces sérotypes n'étant pas cultivés dans des conditions de limitation en fer en présence d'un chélateur de fer ; et
c) un support pharmaceutiquement acceptable.

2. Composition de vaccin suivant la revendication 1, dans laquelle le ou les extraits capsulaires de cultures comprenant un ou plusieurs sérotypes sont préparés à partir des sérotypes de 1, 5 et 7, ou à partir des sérotypes 1 et 7, ou bien à partir des sérotypes 1 et 5 de A. pleuropneumoniae.

3. Composition de vaccin suivant la revendication 1 ou 2, dans laquelle l'extrait capsulaire du premier sérotype est préparé à partir du sérotype 5 de A. pleuropneumoniae.

4. Composition de vaccin suivant la revendication 1, 2 ou 3, dans laquelle le chélateur de fer consiste en 2,2-dipyridyle.

5. Utilisation d'une composition de vaccin suivant l'une quelconque des revendications précédentes dans la production d'un médicament destiné à être utilisé dans la vaccination des porcs contre une infection par A. pleuropneumoniae.

6. Utilisation suivant la revendication 5, dans laquelle le médicament doit être administré en une quantité, efficace du point de vue immunostimulateur, de la composition.
